# EUROPÄISCHE PATENTSCHRIFT

(11) **EP 0 361 113 B1**
(45) Veröffentlichungstag und Bekanntmachung des Hinweises auf die Patenterteilung: **07.07.1993**
(21) Anmeldenummer: 89115912.1
(22) Anmeldetag: 29.08.1989
(51) Int. Cl.: C07C 311/16, C07C 311/17, C07C 311/19, C07D 257/04, A61K 31/18

(54) **Sulfonamidoalkyl-cyclohexan-Verbindungen, Verfahren zu ihrer Herstellung sowie Arzneimittel**
Sulfonamidoalkyl cycloalkane compounds, processes for their production and pharmaceutical preparations
Composés sulfonamidoalkylcycloalcaniques, procédés pour leur préparation et compositions pharmaceutiques

(30) Priorität: 31.08.1988 DE 3829455
(43) Veröffentlichungstag der Anmeldung: 04.04.1990
(73) Patentinhaber: BOEHRINGER MANNHEIM GMBH, 68298 Mannheim (DE)
(72) Erfinder: Witte, Ernst-Christian, Dr. rer. nat., D-6800 Mannheim 1 (DE); Stegmeier, Karlheinz, Dr. rer. nat., D-6148 Heppenheim (DE); Dörge, Liesel, Dr. med., D-6840 Lampertheim (DE)

(56) Entgegenhaltungen:
- EP-A- 0 031 954
- EP-A- 0 221 344
- EP-A- 0 239 907
- EP-A- 0 253 321
- DE-A- 3 541 854
- DE-A- 3 631 824
- DE-A- 3 642 105

## Beschreibung

Die vorliegende Erfindung betrifft neue Sulfonamide der allgemeinen Formel I 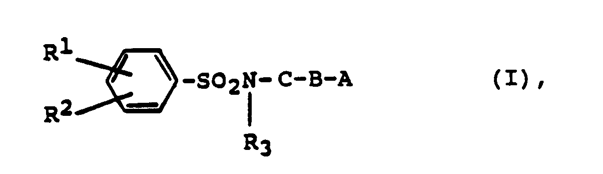
in welcher
- R₁ und R₂: gleich oder verschieden sein können und jeweils Wasserstoff, ein Halogenatom, eine C₁-C₆-Alkyl-, Trifluormethyl-, Cyan-, Carboxyl-, Alkoxycarbonyl-, Aminocarbonyl-, N-Alkyl-aminocarbonyl-oder N,N-Dialkylaminocarbonylgruppe oder für den Fall, daß R₁ und R₂ zueinander o-ständige Alkylgruppen darstellen, R₁ und R₂ zusammen mit den beiden C-Atomen, an die sie geknüpft sind, einen gesättigten oder ungesättigten C₅-C₇-Alkylenring bilden,
- R₃: ein Wasserstoff, eine Alkylgruppe mit 1-6 C-Atomen, eine Acylgruppe, einen Phenylalkyl- oder Phenylalkenylrest darstellt, wobei der Phenylteil durch Halogen, Alkyl oder Trifluormethyl substituiert sein kann,
- C: die Gruppe -(CH₂)ₘ- mit m = 0 bis 3 oder eine verzweigte C₂-C₅-Alkylengruppe bedeutet, wobei eine Methylengruppe -CH₂- der Gruppe C durch ein Sauerstoffatom, Schwefelatom oder durch eine Hydroxymethylengruppe -CHOH- oder Carbonylgruppe -CO- ersetzt sein kann,
- B: 1,2-, 1,3-, 1,4-Cyclohexyliden, 1,2- oder 1,3-Cyclopentyliden symbolisiert,
- A: eine C₁-₆-Alkylkette, eine Hydroxy-C₁₋₆-alkylgruppe, Carboxy-, Carboxy-C₁-₆-alkyl-, Carboxy-C₁-C₆-alkoxy-, Tetrazolyl-, Tetrazolyl-C₁-C₆-alkyl, Tetrazolyl-C₁-C₆-alkoxy-, Tetrazolylaminocarbonyl-C₁-C₆-alkyl- oder eine Tetrazolylaminocarbonyl-C₁-C₆-alkyloxy-Gruppe oder einen Rest D-R⁴ bedeutet, in dem D eine -CO- oder -CHOH- Gruppe und R⁴ Wasserstoff, einen C₁-C₅-Alkyl-, Hydroxy-C₁-C₅-alkyl-, Carboxy-, Carboxy-C₁-C₅-alkyl-, Tetrazolyl- oder Tetrazolyl-C₁-C₅-alkylrest darstellt, wobei in allen Fällen, in denen A eine Carboxylgruppe enthält, diese durch die Hydroxamsäuregruppe -CO-N(OH)R₅ ersetzt sein kann, in der R₅ ein Wasserstoffatom oder eine C₁-C₅-Alkylgruppe bedeutet, und für den Fall, daß A eine Carboxy- und eine Hydroxygruppe enthält, gegebenenfalls deren Lactone,
sowie deren pharmakologisch verträgliche Salze, Ester und Amide und optisch aktive Formen und cis- und trans-Isomere.

Aus EP-A-0,031,954 sind Phenylcarbonsäuren bekannt, die in 4-Stellung durch Sulfonamido-alkyl-Gruppen substituiert sind. Ähnliche Verbindungen, bei denen die Carbonsäuregruppe durch substituierte Alkyl-, Alkenyl- oder Alkylcarbonylgruppen ersetzt ist, sind in EP-A-221,344 beschrieben. Ferner sind aus EP-A-0,239,907 Phenoxyalkylcarbonsäure-Derivate bekannt, in denen die Sulfonamidoalkylgruppe in ortho- oder meta-Stellung zur Phenoxyalkylcarbonsäure-Gruppe steht.

In DE-A-364 2105 werden Aminomethyl-5,6,7,8-tetrahydronaphthyl-oxyessigsäuren der allgemeinen Formel 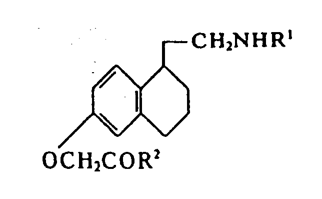
in welcher
- R¹: für SO₂R³ steht, wobei
- R³: für Aryl oder substituiertes Aryl steht,
- R²: für Hydroxy, Alkoxy, Phenoxy, Benzoxy oder NR⁴R⁵ steht, wobei
- R⁴ und R⁵: gleich oder verschieden sind und jeweils für Wasserstoff oder Alkyl stehen oder einer der Reste R⁴ oder R⁵ für Benzylsteht, offenbart.

Außerdem werden in EP-A-253 321 Benzolsulfonamido-indanylverbindungen der allgemeinen Formel 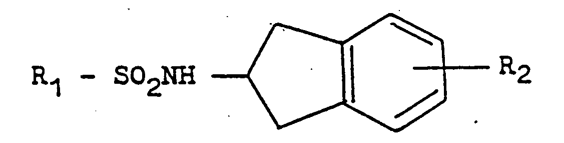
in der
R₁ eine gegebenenfalls durch ein Halogenatom, eine Methyl-oder Methoxygruppe mono-oder disubstituierte Phenylgruppe, wobei die Substituenten gleich oder verschieden sein können, und
R₂ eine gegebenenfalls über eine geradkettige oder verzweigte Alkylengruppe oder über eine Alkenylengruppe gebundene Hydroxycarbonyl-oder Alkoxycarbonylgruppe, wobei jeweils eine Methylengruppe der vorstehend erwähnten Alkylen-oder Alkenylenreste, welche mit dem lndanylrest verknüpft sein muß, durch eine Hydroxymethylen-oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe substituiert sein kann, oder eine 4,5-Dihydro-pyridazin-3(2H)-on-6-yl-oder Pyridazin-3(2H)-on-6-yl-gruppe. welche in 4-oder 5-Stellung durch eine Alkylgruppe und/oder in 4-Stellung durch eine Alkoxycarbonylgruppe substituiert sein können bedeuten, deren Enantiomere und deren Salze mit anorganischen oder organischen Basen, falls R₂ eine Hydroxycarbonylgruppe enthält, offenbart.

Es wurde nun überraschenderweise gefunden, daß man Verbindungen mit neuen wertvollen pharmakologischen Eigenschaften erhält, wenn man bei den aus dem oben genannten Stand der Technik bekannten Verbindungen die vorhandene Phenylgruppe durch eine Cyclohexyliden- oder Cyclopentyliden-Gruppe ersetzt.

Die neuen Verbindungen der allgemeinen Formel I zeigen eine ausgezeichnete antagonistische Wirkung gegenüber Thromboxan A₂ sowie gegen Prostaglandin-endoperoxide und sind den aus dem Stand der Technik bekannten Verbindungen wirkungsmäßig überlegen. Sie inhibieren die Aggregation von Blutplättchen und verhindern die Konstriktion der glatten Muskulatur sowie die Bronchokonstriktion. Sie sind außerdem wertvolle Heilmittel zur Behandlung pathologischer Veränderungen der Nierenfunktion.

Diese Eigenschaften machen sie zu wertvollen Heilmitteln zur Behandlung z.B. von cardiovaskulären Erkrankungen und von Asthma und zur Prophylaxe einer Schocklunge. Sie können weiterhin verwendet werden bei Organtransplantationen und Nierendialyse und sind geeignet, Rezidive bei Magengeschwüren zu verhindern. Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können z.B. gegen cerebrale ischaemische Zustände eingesetzt werden.

Sind R¹, R², R³ oder A niedere Alkylgruppen, so seien darunter unverzweigte oder verzweigte Gruppen mit 1-6 C-Atomen verstanden. Bevorzugt sind Methyl, Butyl- und n-Hexyl.

Für den Fall, daß R₁ und R₂ zusammen einen Ring bilden, so kommt bevorzugt ein Sechsring in Frage. Besonders bevorzugt ist in diesem Sinne der Fall, in dem R₁ und R₂ zusammen mit dem Ring, an den sie gebunden sind, einen alpha-oder β-Naphthyl- oder Tetrahydronaphthylrest darstellen, der gegebenenfalls durch die unter R₁ definierten Gruppen, insbesondere durch Halogen substituiert ist.

Als Halogenatome kommen Fluor, Chlor und Brom in Frage. Bevorzugt sind Chlor und Brom.

Als Tetrazolylgruppe kommt insbesondere die 1H-Tetrazol-5-yl-gruppe in Frage. Als Phenylalkylgruppen R³ kommen solche mit 1-3 C-Atomen im Alkylenteil infrage, wobei der Phenylrest insbesonders durch Halogen substituiert ist. Unter Phenylalkenyl R³ sind Gruppen zu verstehen, deren Alkenylenanteil 3-4 C-Atome enthält. Auch hier ist der Phenylteil gegebenenfalls durch Halogen substituiert.

Die Acylreste für R₃ leiten sich von aliphatischen Carbonsäuren mit 2-6 C-Atomen und von araliphatischen und aromatischen Carbonsäuren ab. Beispielsweise kommen für R₃ die Acetyl-, Formyl- oder Benzoylgruppe in Frage.

Für C kommt bevorzugt die Bedeutung -(CH₂)ₘ mit m = 0-3 in Frage. B ist vorzugsweise eine Cyclohexylidengruppe, wobei die Substituenten A und C in cis- oder trans-Stellung zueinander stehen können, und die Substitution vorzugsweise in 1,4- und 1,3-Stellung erfolgt.

A wird definiert als Alkylkette mit 1-6 C-Atomen, oder als Alkylkette mit 1-6 C-Atomen, die endständig eine Hydroxyfunktion trägt. A soll außerdem eine Carboxylgruppe, eine Tetrazolylgruppe oder C₁₋₅-Alkylkette mit endständiger Carboxyl- oder Tetrazolylgruppe dardarstellen. Die eben definierten Alkylketten können jeweils durch eine ein Sauerstoffatom enthaltende Funktion, d.h. durch eine Hydroxy- oder eine Oxogruppe, substituiert sein.

Außerdem kann A bevorzugt einen Carboxy-C₁-C₅-alkyloxy-oder Tetrazolyl-C₁-C₅-alkyloxyrest bedeuten, insbesondere jedoch eine Oxyessigsäuregruppe und eine Tetrazolylmethyloxygruppe.

Bevorzugt sind die nachstehend aufgeführten Gruppen A:
1. A = eine unverzweigte oder verzweigte gesättigte Alkylkette, insbesondere eine solche der Formel

   -(CH₂)ₚ₋₁CH₃,

   in welcher p die Zahlen 1-6 darstellt.
2. A = eine unverzweigte oder verzweigte gesättigte Alkylkette, die endständig eine Carboxylfunktion oder eine Tetrazolylgruppe trägt, insbesondere solche der Formeln

   -(CH₂)ₚ₋₁-COOH,

   und

   -(CH₂)ₚ₋₁-Tetrazolyl

   in welchen p die Zahlen 1-6 darstellt.
3. A = eine unverzweigte oder verzweigte gesättigte Alkylkette, die endständig eine Hydroxygruppe trägt, insbesondere solche der Formel

   -(CH₂)ₚ-OH

   in welcher p die Zahlen 1 bis 6 darstellen kann.
4. A = eine Gruppe -D-R₄, wobei D die Carbonyl-Gruppe -CO- und
   a) R⁴ = ein unverzweigter oder verzweigter gesättigter Alkylrest mit 1-5 C-Atomen darstellt. Bevorzugt ist

      A = -CO-(CH₂)ₚ₋₂-CH₃

      wobei p die Zahlen 2 bis 6 darstellt.
   b) R⁴ = ein unverzweigter oder verzweigter gesättigter Alkylrest mit 1-5 C-Atomen, der endständig eine Hydroxygruppe trägt.
      Bevorzugt sind Verbindungen mit der Gruppe

      A = -CO-(CH₂)ₚ₋₁-OH

      mit p = 3 bis 6.
   c) R⁴ = eine unverzweigte oder verzweigte Alkylkette mit endständiger Carboxyl- oder Tetrazolylgruppe, wobei bevorzugt sind Verbindungen mit den Gruppen

      A = -CO-(CH₂)ₚ₋₂-COOH

      und

      A = -CO-(CH₂)ₚ₋₂-Tetrazolyl

      mit p = 3 bis 6, insbesondere aber
      p = 4 bis 6
5. A = eine Gruppe D-R₄, wobei D die Gruppe -CHOH- und
   R⁴ die oben angegebenen Bedeutungen hat. Bevorzugt sind folgende Gruppen
   A:

   a) A = -CHOH-(CH₂)ₚ₋₂-CH₃

   mit p = 2 bis 6

   b) A = -CHOH-(CH₂)ₚ₋₁-OH

   mit p = 3 bis 6 und

   c) A = -CHOH-(CH₂)ₚ₋₂-COOH

   mit p = 4 bis 6

   d) A = -CHOH-(CH₂)ₚ₋₂-Tetrazolyl

   mit p = 4 bis 6
6. A = eine Gruppe -O-(CH₂)_{q}-COOH, wobei q die Zahlen 1-5 darstellen kann.

Besonders bevorzugt sind die folgenden Bedeutungen der Reste R¹ - R³, A, B und C:
- R₁ oder R₂: ein Wasserstoff-, Chlor- oder Bromatom und Methyl, n-Butyl und n-Hexyl, Trifluormethyl, Cyan oder Aminocarbonyl. Bevorzugt für R₁ und R₂ ist jedoch das Wasserstoffatom, bzw. die monosubstituierten Derivate, vorzugsweise in 4-Stellung des Phenylrings.
- R₃: Wasserstoff, eine Methyl-, n-Butyl oder eine n-Hexylgruppe; Acetyl, n-Hexanoyl oder Benzoyl; Benzyl; Phenethyl oder Cinnamyl, wobei deren Phenylteil durch Halogen, insbesondere durch Chlor substituiert sein kann.
- C: die Gruppe -(CH₂)ₘ- mit m = 2 oder 3, oder die Gruppe -O-CH₂-, -CH₂-CH₂-O-, -CH₂-CO-, -CH₂-CHOH-.
- B: 1,4-Cyclohexyliden, wobei die Substituenten cis-oder trans-ständig stehen.
- A: eine Ethyl-, Butyl- oder eine Hexylgruppe, oder auch eine Carboxyl- oder 1H-Tetrazol-5-yl-gruppe bzw. eine durch eine dieser beiden Gruppen endständig substituierte Alkylkette. In diesem Falle sind besonders bevorzugt: A = Carboxyl, 1H-Tetrazol-5-yl, Carboxymethyl, Carboxypropyl und Carboxybutyl, bzw. deren 1H-Tetrazol-5-yl-analoga.
Besonders bevorzugte Gruppen A wie unter Punkt 4 a angegeben sind z.B. Acetyl, Butyryl und Hexanoyl, des Typs 4 b die Gruppe 4-Hydroxybutanoyl, und des Typs 4 c die Gruppe 3-Carboxy-propanoyl.
Für den Typ 5 seien als besonders bevorzugte Gruppen A genannt:
Für den Typ 5 a die Gruppen 1-Hydroxyethyl, 1-Hydroxybutyl und 1-Hydroxyhexyl; für den Typ 5 b die Gruppe 1,4-Dihydroxybutyl, und für den Typ 5 c die Gruppe 1-Hydroxy-3-carboxypropyl und 1-Hydroxy-3-(1H-tetrazol-5-yl)propyl. Für den Typ 6 kommt als Oxyalkylcarbonsäure besonders bevorzug die Oxyessigsäure mit q=1 in Frage.
Für den Fall, daß A eine endständig durch eine Hydroxygruppe substituierte Alkylkette darstellt, seien besonders bevorzugt die Gruppen 2-Hydroxyethyl, 4-Hydroxybutyl und 6-Hydroxyhexyl.

Für den Fall, daß die Gruppe A Carboxylgruppen enthält, werden auch deren Salze, Ester und Amide beansprucht und für den Fall, daß A sowohl eine Carboxylgruppe als auch eine Hydroxygruppe enthält, auch die "inneren Ester", die Lactone.

Unter den Oxyalkylcarbonsäuren sind besonders bevorzugt die Oxyessigsäuren und unter den Tetrazolyl-C₁-C₅-alkyloxyverbindungen solche mit der (1H-Tetrazolyl-5-yl)methyloxy-gruppe.

Als Ester kommen solche mit niederen einwertigen Alkoholen (wie z.B. Methanol oder Ethanol) oder mit mehrwertigen Alkoholen (wie z.B. Glycerin) infrage, es seien aber auch solche Alkohole eingeschlossen, die noch andere funktionelle Gruppen enthalten, wie z.B. Ethanolamin.

Als Amide seien besonders bevorzugt die Tetrazolyl-5-amide, beispielsweise die Essigsäure-tetrazolyl-amide, d.h. A = CH₂-CO-NH-tetrazolyl. Als ebenfalls bevorzugte Amide sind die Hydroxamsäuren zu nennen, beispielsweise die Cyclohexyloxyessigsäure-N-hydroxylamide, d. h. A = O-CH₂-CONHOH.

Für den Fall, daß eine Verbindung der allgemeinen Formel I asymmetrische Kohlenstoffatome enthält, so seien sowohl die reinen optischen Isomere als auch deren Gemische/ - Racemate umfaßt. Enthält das Molekül Doppelbindungen, so werden hier die reinen E- und Z-Isomere sowie auch deren Gemische beansprucht. Bezüglich der Substituenten am Cyclohexylring kommen sowohl die cis- als auch trans-Isomeren in Frage.

Die Herstellung der Verbindungen der allgemeinen Formel I ist dadurch gekennzeichnet, daß man
a) ein Amin der allgemeinen Formel II 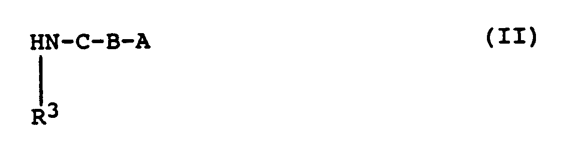 in welcher R³, C, B und A die angegebene Bedeutung haben,
   in an sich bekannter Weise mit einer Sulfonsäure der allgemeinen Formel III 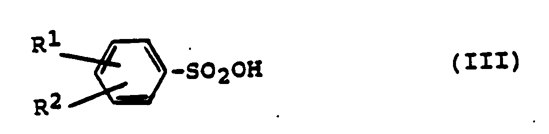 in welcher R¹ und R² hier und in allen folgenden Beispielen die oben angegebene Bedeutung haben, bzw. mit einem Derivat derselben umsetzt.
   Anstelle der freien Amine II kann man auch deren Salze einsetzen.
b) ein Sulfonamid der allgemeinen Formel IV 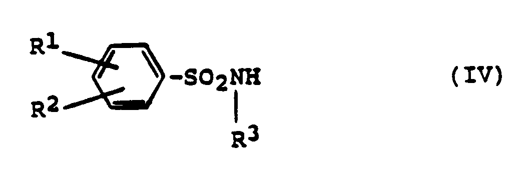 mit einer Verbindung der allgemeinen Formel V

   X-C-B-A (V)

   zur Umsetzung bringt. X soll hier und in allen folgenden Erläuterungen eine reaktive Gruppe symbolisieren.
   Für den Fall, daß A Hydroxygruppen enthält, verwendet man in manchen Fällen vorteilhaft eine solche Verbindung V, die an der Stelle der Hydroxygruppe eine Carboxygruppe oder eine Esterfunktion (oder ggf. beide) enthält. Diese Gruppe wird im Anschluß an die erfolgte Reaktion zwischen IV und V zur Hydroxylfunktion reduziert.
c) Zur Herstellung von Verbindungen I, in denen R³ die oben angegebene Bedeutung hat, jedoch kein Wasserstoffatom darstellt, kommt man auch durch nachträgliches Einführen von R³, indem man eine Verbindung der allgemeinen Formel I a 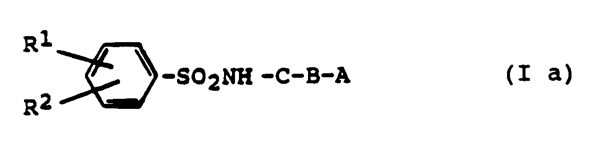 mit einer Verbindung der allg. Formel VI

   R³-X (VI)

   in der R³ kein Wasserstoffatom darstellt, umsetzt.
   Bei Anwesenheit von Hydroxygruppen im Rest A gilt das unter b) Gesagte sinngemäß.
d) Zur Einführung von Hydroxygruppen in den Rest A kommen folgende Verfahren infrage:
   1. Reduktion einer Carbonylgruppe
   2. Reduktion einer Carbonsäure- oder einer Carbonsäureester-funktion
   3. Gleichzeitige Reduktion beider.
e) Zur Herstellung von Verbindungen I, in denen A = -O-(CH₂)_{q}-COOH, eignet sich auch die Umsetzung von substituierten Cycloalkanolen der allgemeinen Formel VII 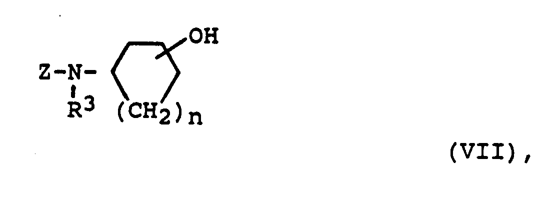 in welcher Z und R³ die Funktion einer Schutzgruppe für die Aminogruppe haben und n die Zahlen 1 oder 2 bedeutet, mit Halogencarbonsäuren der allgemeinen Formel VIII

   Hal-(CH₂)_{q}-COOH (VIII),

   und gegebenenfalls anschließenden Abspaltung der Schutzgruppe und Umsetzung der Aminogruppe mit einer Sulfonsäure der Formel III.
f) Zur Herstellung von Verbindungen I, in denen A eine 1H-Tetrazol-5-yl-Gruppe enthält, geht man von den entsprechenden Nitrilen aus, welche mit Stickstoffwasserstoffsäure zu den Tetrazolen zyklisieren.

Als reaktive Derivate der Sulfonsäuren III kommen insbesondere die Halogenide sowie die Ester infrage. Die Umsetzungen der Sulfonsäurehalogenide mit Verbindungen der allgemeinen Formel II erfolgen zweckmäßig unter Zusatz eines säurebindenden Mittels, wie z.B. Alkaliacetat, Natriumhydrogencarbonat, Natriumcarbonat, Natriumphosphat, Calciumoxid, Calciumcarbonat oder Magnesiumcarbonat. Diese Funktion können aber auch organische Basen wie z.B. Pyridin oder Triethylamin übernehmen, wobei als inertes Lösungsmittel z.B. Ether, Benzol, Methylenchlorid, Dioxan oder ein Überschuß des tertiären Amins dient. Die Amine der allgemeinen Formel II können auch in Form ihrer Säureadditionssalze, wie z. B. Hydrochlorid eingesetzt werden.

Bei Einsatz anorganischer Säurebinder verwendet man als Reaktionsmedium z.B. Wasser, wässriges Ethanol oder wässriges Dioxan.

Zur Alkylierung der Sulfonamide IV verwendet man Verbindungen V, in denen X ein Halogenatom wie Chlor oder Brom darstellt, bevorzugt aber solche, in denen X eine Arylsulfonyloxygruppe darstellt, als Alkylierungsmittel dienen also bevorzugt Arylsulfonsäurealkylester, eine Methode, die in ihrer Anwendung auf Sulfonsäureamide z.B. bei Klamann et al., Monatshefte für Chemie Bd. 83 (1952), S. 871 beschrieben ist. Die Umsetzungen erfolgen in alkalischem Milieu. Bevorzugt ist als Reaktionsmedium heiße, konzentrierte Sodalösung. Ist dagegen X ein Halogenatom, so setzt man ein Alkalisalz des Sulfonamids IV, z.B. das Natriumsalz, mit V (X = Chlor oder Brom) in polaren Lösungsmitteln wie z.B. Dimethylformamid um. Um eine Disubstitution des Sulfonamids IV zu vermeiden, wird IV zweckmäßig im Überschuß eingesetzt.

Soll im Anschluß an die Sulfonamidbildung eine Gruppe R³ eingeführt werden, so geschieht dies durch Umsetzen einer Verbindung I, in der R³ = H bedeutet, mit einem Säurehalogenid, wenn R³ eine Acylgruppe darstellt.
Für alle anderen Bedeutungen von R³ setzt man ein Halogenid (Chlorid oder Bromid) der allg. Formel VI ein, wobei unter den oben angegebenen Bedingungen gearbeitet wird.

Die Acylierung des Sulfonamids erfolgt in einem inerten Lösungsmittel wie Ether oder Methylenchlorid, als säurebindendes Agens verwendet man bevorzugt organische Basen wie Pyridin oder Triethylamin.
Die im Anschluß an die Sulfonamidbildung und ggf. nach Einführung einer Gruppe R³ möglichen Umwandlungen im Rest A lassen sich wie folgt beschreiben.

Zur Umwandlung einer Carbonylgruppe in eine Hydroxygruppe sind hier alle gängigen Verfahren einsetzbar. Bevorzugt ist die Reduktion mit komplexen Borhydriden, z.B. mit Natriumborhydrid, wobei protische Lösungsmittel wie Wasser, (wäßrige) Alkohole oder wäßriges Dioxan als Reaktionsmedium dienen. Bei Abwesenheit anderer reduzierbarer Gruppen kann die Reduktion auch mit komplexen Aluminiumhydriden wie LiAlH₄ durchgeführt werden, wobei hier aprotische Lösungsmittel wie Ether, THF oder Dioxan als Reaktionsmedium dienen. Die Carbonylreduktion kann aber auch mit katalytisch angeregtem Wasserstoff erfolgen, z.B. mit H₂/Raney-Nickel oder durch Umsetzen mit Nickel-Aluminium-Legierung in wäßrigem Alkali.

Zur Reduktion der Carboxylfunktion sind alle gängigen Reduktionsmittel geeignet, z.B. komplexe Hydride wie Lithiumaluminium-hydrid oder Boran-addukte wie z.B. BH₃ · THF. Die Reduktion kann aber auch vorteilhaft durch Reduzieren eines Derivates der Carbonsäure, z.B. eines gemischten Anhydrids aus der Carbonsäure und einem Kohlensäurehalbester, erfolgen. Als Reduktionsmittel verwendet man hier bevorzugt komplexe Borhydride wie z.B. NaBH₄ in protischen Lösungsmitteln.

Zur Reduktion geeignete Derivate der Carbonsäuren sind z. B. auch deren Ester, welche sich nach literaturüblichen Methoden zu primären Alkoholen umsetzen lassen. Bevorzugte Reduktionsmittel sind auch hier komplexe Aluminiumhydride wie z.B. Lithiumalanat.

Soll die Carboxylfunktion reduziert werden, ohne daß eine gleichzeitig in A befindliche Oxogruppe mitreduziert wird, so ist letztere z.B. durch Ketalisierung intermediär zu schützen. Solche Hydroxy-ketone sind auch darstellbar, indem man sowohl die Ketogruppe als auch die Carboxylfunktion reduziert (wobei man die ebenfalls beanspruchten Diole erhält) und anschließend die sekundäre Hydroxyfunktion selektiv zur Ketofunktion oxidiert. Hierzu geeignet ist z.B. aktives Mangandioxid.

Die Veretherung von Cycloalkanolen der Formel VII mit Halogencarbonsäuren erfolgt in Gegenwart von Alkoholat bildenden Agenzien, z.B. von Natriumhydrid. Lithiumbutyl, bevorzugt aber unter Phasentransferbedingungen, d.h. in einem Gemisch aus Alkalilauge, einem geeigneten organischen Solvens wie z.B. Methylenchlorid, und in Gegenwart eines quaternären Ammoniumsalzes wie z.B. Tetran-butyl-ammoniumbromid. Die darauf folgende Abspaltung von Schutzgruppen Z und R³ der allg. Formel VII kann natürlich entfallen, wenn Z schon eine Gruppe 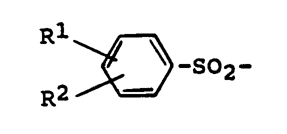
und R³ eine der beanspruchten Gruppen außer H darstellt.

Zur Herstellung von Verbindungen I, in denen A eine 1H-Tetrazol-5-yl-Gruppe darstellt, geht man von Nitrilen aus, die mit Stickstoffwasserstoffsäure oder, bevorzugt, mit einem ihrer Salze zur Tetrazolgruppe zyklisiert werden:

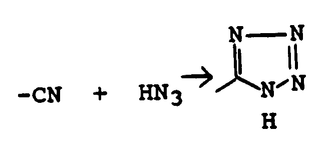

Die Salze der Stickstoffwasserstoffsäure, die in dem erfindungsgemäßen Verfahren mit Nitrilen zur Reaktion gebracht werden, können beispielsweise Alkalimetallsalze sein, wie Lithiumazid, Natriumazid und Kaliumazid, Erdalkalimetallsalze wie Magnesiumazid, Calziumazid und Strontiumazid, oder Metallsalze wie Aluminiumazid, Zinnazid, Zinkazid und Titanazid, Salze mit organischen Basen wie Ammoniumazid und Aniliniumazid und andere. In einigen Fällen ist es zweckmäßig, nicht die reinen Azide einzusetzen, sondern sie im Gemisch z.B. mit Ammoniumchlorid oder mit einem Alkylammoniumchlorid, oder auch in Kombination mit einer Lewis-Säure wie Aluminiumchlorid, Zinnchlorid, Zinkchlorid oder Titantetrachlorid zur Reaktion zu bringen.

Die Stickstoffwasserstoffsäure oder ihre Salze sowie die Lewis-Säure oder Ammoniumchlorid bzw. Alkylammoniumchlorid, die in Kombination mit den Alkaliaziden benützt werden, werden im 1- bis 10-molaren Überschuß, bezogen auf die Nitrile eingesetzt.

Zur Herstellung von Salzen mit pharmakologisch verträglichen organischen oder anorganischen Basen, wie z B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Die als Ausgangsmaterial für die Herstellung von Verbindungen der allgemeinen Formel I benötigten Amine der allgemeinen Formel II, in der B Cyclohexyliden bedeutet, werden zweckmäßig durch Hydrieren der aromatischen Analoga IX 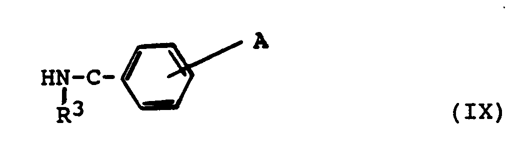
dargestellt. Die Verbindungen der allgemeinen Formel IV und VII bzw. deren Vorstufen sind aus EP-A-0,031,954, EP-A-0,221,344 und EP-A-0,239,907 bekannt. Die Hydrierung erfolgt in Gegenwart von Metallkatalysatoren wie Platin, Ruthenium oder Rhodium, ggf. in Form ihrer Oxide (z.B. RuO₂) bei erhöhtem Druck, z.B. zwischen 50 und 150 bar, und bei erhöhter Temperatur, z.B. zwischen 70 und 100°C. Als Lösungsmittel sind bevorzugt niedere aliphatische Carbonsäuren wie z.B. Essigsäure, oder insbesondere Alkohole wie z.B. Ethanol.

Bedeutet in der Formel IX A einen Rest -CO-R⁴, so reduziert man zur Herstellung der Amine II zunächst die Carbonylverbindung zum Carbinol und hydriert anschließend den aromatischen Kern unter schonenden Bedingungen (bei ca. 50°C in Gegenwart von PtO₂ oder RuO₂ in Ethanol). Danach wird die Carbinolgruppe wiederum zur Oxogruppe oxidiert.

Zur Herstellung von Verbindungen II, in denen A beispielsweise die Bedeutung -COCH₂CH₂COOH hat, eignet sich die Umsetzung gemäß dem folgenden Formelschema:

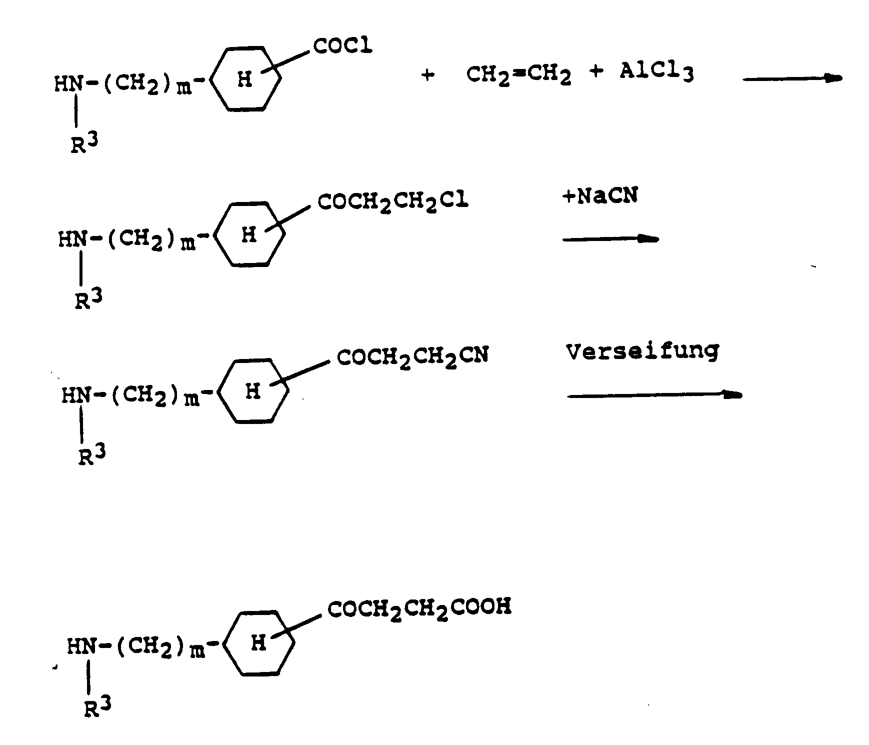

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hifsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Poylmere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gegebenenfalls gleichzeitig durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Die nachfolgenden Beispiele zeigen die praktische Realisierung des Erfindungsgedankens. Außer den in den Beispielen beschriebenen Verbindungen kommen die folgenden Verbindungen in Frage:
1. 3-[2-(4-Chlor-benzolsulfonylamino)ethyl]cyclohexyloxyessigsäure; Schmp. 105-106°C
2. 3-[2-(4-Chlor-benzolsulfonylamino)ethyl]cyclohexyloxyessigsäure-(tetrazol-5-yl-amid); Schmp. 185-188°C
3. cis-4-[2-(4-Chlor-benzolsulfonylamino)ethyl]cyclohexylessigsäure
4. trans-4-[2-(4-Chlor-benzolsulfonylamino)ethyl]cyclohexylessigsäure
5. 4-<4-[2-(4-Chlor-benzolsulfonylamino)ethyl]cyclohexyl>-4-oxo-butansäure
6. 4-<4-[2-(4-Chlor-benzolsulfonylamino)ethyl]cyclohexyl>-4-hydroxy-butansäure
7. 1-<4-[2-(4-Chlor-benzolsulfonylamino)ethyl]cyclohexyl>-1.4-butandiol
8. 1-Butanoyl-4-[2-(4-Chlor-benzolsulfonylamino)ethyl]cyclohexan
9. 1-<4-[2-(4-Chlor-benzolsulfonylamino)ethyl]cyclohexyl>-1-hydroxy-butan
10. 1-<4-[2-(4-Chlor-benzolsulfonylamino)ethyl]cyclohexyl>butan
11. 1-[2-(4-Chlor-benzolsulfonylamino)ethyl]-3-(1H-tetrazol-5-yl-methylenoxy)cyclohexan
12. 1-[2-(4-Chlor-benzolsulfonylamino)ethyl]4-(1H-tetrazol-5-yl-methyl)cyclohexan; Schmp. 125-128°C
13. N-(4-Chlor-benzolsulfonyl)-0-[4-(2-hydroxyethyl)cyclohexylmethyl]hydroxylamin
14. [2-(4-Chlor-benzolsulfonylamino)ethyl]-[4-(carboxymethyl)cyclohexyl]ether
15. 4-[2-(4-Chlor-benzolsulfonylamino)-1-oxo-ethyl]cyclohexancarbonsäure
16. 4-[2-(4-Chlor-benzolsulfonylamino)-1-hydroxy-ethyl] cyclohexancarbonsäure
17. 4-[2-(4-Chlor-benzolsulfonylamino)ethyl]cyclohexyloxyessigsäure-hydroxylamid
18. 4-[2-(4-Chlor-benzolsulfonylamino)-ethyl]cyclohexylessigsäureamid; Schmp. 140-141°C

### Beispiel 1

### 4-[2-(Benzolsulfonylamino)ethyl]-cyclohexyloxyessigsäure

### a) 4-(2-Acetamino-ethyl)cyclohexyloxyessigsäure-ethylester

Man hydriert ein Gemisch aus 20.0 g (75 mmol) 4-[2-Acetamino-ethyl]phenoxyessigsäure-ethylester, 250 ml Ethanol und 0.7 g Ruthenium-IV-Oxid 12 Stdn. bei 70°C und 60 bar, filtriert und dampft das Filtrat i. Vak. ein. Ausb. quantitativ, farbloses Öl.

### b) 4-(2-Amino-ethyl)cyclohexyloxyessigsäure-ethylester

Ein Gemisch aus 17.0 g (70 mmol) des nach a) erhaltenen Ethylesters und 100 ml 2 N HCl wird 8 Stdn. auf Rückflußtemperatur gehalten. Dann dampft man ein, gibt zum trocknen Rückstand 50 ml Ethanol und eine Spatelspitze p-Toluolsulfonsäure und hält 6 Stdn. auf Rückflußtemperatur. Anschließend wird eingedampft. Ausb. 17.0 g (91 % d.Th.), farbloses Öl.
1H-NMR (300 MHz, d₆-DMSO): δ = 1.21 (t, J = 7Hz, 3H, OCH₂CH₃); 3.97 (s, 2H, OCH₂); 4.10 (q, 7Hz, 2H, OCH₂CH₃)

### c) 4-[2-(Benzolsulfonylamino)ethyl]cyclohexyloxyessigsäure-ethylester

Zu einer eiskalten Lösung aus 8.5 g (30 mmol) 4-(2-Amino-ethyl)cyclohexyloxyessigsäure-ethylester und 75 ml Pyridin tropft man unter Rühren 5.5 g (30 mmol) Benzolsulfonylchlorid, dann läßt man eine Std. bei Raumtemperatur und eine Std. bei 60°C nachreagieren, kühlt ab und gießt in ein Eis-Salzsäuregemisch. Dieses wird mit Methylenchlorid extrahiert. Man wäscht die Methylenchloridphase zweimal mit verd. HCl und zweimal mit Wasser, trocknet über MgSO₄ und dampft ein. Ausb. 10.6 g (89 % d.Th.), farbloses Öl.

### d) Titelverbindung

Ein Gemisch aus 7.4 g (20 mmol) des nach c) erhaltenen Ethylesters, 85 ml 1 N NaOH und 85 ml Methanol wird 8 Stdn. auf Rückflußtemperatur gehalten. Dann destilliert man das Methanol ab und extrahiert die wäßrige Phase zweimal mit Ether. Nun wird angesäuert und mit Methylenchlorid extrahiert. Man trocknet die versetzt die freie Säure (5.0 g, Ausb. 69 % d.Th.) mit der berechneten Menge NaHCO₃ in wäßr. Lösung und engt ein. Digerieren mit einem Gemisch aus 1 Vol. Ether + 1 Vol. Ethanol liefert das kristalline Natriumsalz. Ausb. 72 % d.Th., Schmp. 135-136°C.

### Beispiel 2

### 4-[2-(4-Chlor-benzolsulfonylamino)ethyl]cyclohexan-carbonsäure

### a) 4-(2-Aminoethyl)cyclohexancarbonsäure-ethylester

Man hydriert ein Gemisch aus 15.0 g (65 mmol) 4-(2-Aminoethyl)benzoesäure-ethylester-hydrochlorid, 300 ml Ethanol und 1 g Ruthenium-IV-oxid 10 Stdn. bei 100 bar und 90°C, saugt den Katalysator ab und dampft ein. Das Rohprodukt wird in heißem Essigester gelöst, dann kühlt man im Eisbad ab und fällt die Substanz durch Zugabe von Isohexan aus. Ausb. 11.4 g (74 % d.Th.) Hydrochlorid mit dem Schmp. 114-116°C.

### b) 4-[2-(4-Chlor-benzolsulfonylamino)ethyl]cyclohexancarbonsäure-ethylester

Man rührt ein Gemisch aus 5.5 g (23.3 mmol) 4-(2-Aminoethyl)cyclohexancarbonsäure-ethylester-hydrochlorid, 50 ml Methylenchlorid und 5.9 g (58 mmol) Triethylamin 30 min bei Raumtemperatur, kühlt im Eisbad ab und tropft innerhalb 20 min eine Lösung aus 4.92 g (23.3 mmol) 4-Chlorbenzolsulfonylchlorid und 50 ml Methylenchlorid zu. Nun wird eine Std. bei 20°C nachgerührt, dann zweimal mit kalter 2 N Salzsäure und zweimal mit Wasser extrahiert und die Methylenchloridphase mit Na₂SO₄ getrocknet. Man erhält 8.59 g (98 % d.Th.) Produkt. Farbloses Öl.
1H-NMR (300 MHZ, d₆-DMSO): δ = 2.72-2.82 (m, 2H, NHCH₂)

### c) Titelverbindung (Beispiel 2.1)

Man hält ein Gemisch aus 7.94 g (21 mmol) der nach b) erhaltenen Verbindung, 25 ml 2 N Natronlauge und 20 ml Ethanol drei Stdn. bei 50-60°C und destilliert dann i.Vak. das Ethanol ab. Nach Zugabe von 0.5 N Natronlauge wird ausgeethert und die wäßrige Phase mit 2 N Salzsäure angesäuert. Man nimmt die ölig ausfallende Säure in Ether auf, trocknet mit Na₂SO₄ und dampft ein. Ausbeute 6.52 g (89 % d.Th.), Schmp. 127-128°C (Ethanol).

In analoger Weise wurden hergestellt:

### 2) 4-[2-(4-Chlor-benzolsulfonylamino)ethyl]cyclohexylessigsäure

über folgende Stufen:

### a) 4-(2-Aminoethyl)cyclohexyl-essigsäure-ethylester

durch Hydrieren von 4-(Aminoethyl)phenylessigsäure-ethylester-hydrochlorid i. Ggw. von RuO₂ in Ethanol.
Ausb. 90 % d.Th. Hydrochlorid; Schmp. 108-110°C.

### b) 4-[2-(4-Chlor-benzolsulfonylamino)ethyl]cyclohexylessigsäure-ethylester

durch Umsetzen des nach a) erhaltenen Produktes mit 4-Chlor-benzolsulfochlorid in Pyridin. Ausb. 91 % d.Th.; Schmp. 63-65°C.

### c) Titelverbindung

durch Verseifen des Ethylesters mittels 2 N NaOH in Ethanol.
Ausb. 84 % d.Th. Säure; Schmp. 110-111°C (Ethanol + Wasser 2:1 Vol.).

### 3) 3-[4-(2-Benzolsulfonylamino-ethyl)cyclohexyl]propionsäure

über folgende Stufen:

### a) 3-[4-(2-Acetamino-ethyl)cyclohexyl]propionsäuremethylester

durch Hydrieren von 3-(4-(2-Acetaminoethyl)phenyl]propionsäure-methylester i. Ggw. von RuO₂ in Ethanol.
Ausb. 82 % d.Th., halbkristallines Produkt
1H-NMR (300 MHz, d₆-DMSO). δ = 1.76 (s, 3H, CH₃CO); 2.28 (6. J = 7Hz , 2H, CH₂COOMe); 3.57 (s, 3H, OCH₃).

### b) 3-[4-(2-Amino-ethyl)cyclohexyl]propionsäure

durch Hydrolyse mittels 6 N HCl.
Ausb. 75 % d.Th. Hydrochlorid; Schmp. 215-216°C.

### c) 3-[4-(2-Amino-ethyl)cyclohexyl]propionsäureethylester

durch Begasen einer Ethanol-Lösung der Säure mit Chlorwasserstoff.
Ausb. 81 % d.Th. Hydrochlorid; halbkristalline Masse.

### d) 3-<4-[2-(Benzolsulfonylamino)ethyl]cyclohexyl>-propionsäure-ethylester

durch Umsetzen des nach c) erhaltenen Produktes mit Benzolsulfochlorid in Pyridin.
Ausb. 76 % d.Th.; farbloses Öl.
1H-NMR (300 MHZ, d₆-DMSO): δ = 2.20-2.28 (m, 2H, CH₂COOC₂H₅); 2.70-2.80 (m, 2H, NHCH₂); 7.45 (t, J = 4Hz, 1H, NHSO₂); 7.53-7.81 (m, 4H, arom. Protonen).

### e) Titelverbindung

durch Verseifen des Ethylesters mittels 2 N NaOH in Methanol und Neutralisieren der freien Säure mit NaHCO₃.
Ausb. 68 % d.Th. Natriumsalz; Schmp. 297-301°C.

### 4) 4-[3-(4-Chlor-benzolsulfonylamino)propyl]cyclohexyl-essigsäure über folgende Stufen:

### a) 4-(3-Aminopropyl)cyclohexyl-essigsäure-ethylester

durch Hydrieren von 4-(3-Aminopropyl) phenylessigsäure-ethylester-hydrochlorid an Rhodium in Ethanol.
Ausb. 90 % d.Th., Schmp. 99-103°C

### b) 4-[3-(4-Chlor-benzolsulfonylamino) propyl]cyclohexyl-essigsäure-ethylester

durch Umsetzen des nach a) erhaltenen Produktes mit 4-Chlor-benzolsulfochlorid in Gegenwart von Triethylamin in Methylenchlorid.
Ausb. 95 % d. Th., farbloses Oel
IR 48438, NMR V 3731
¹H-NMR (300 MHz, d₆-DMSO): δ = 1.17 (t, 3H, OCH₂CH₃; (2.12 und) 2.22 (d, J = 7 Hz, 2H, (zus. 2H)
CH₂-COOEt. cis/trans-Gemisch. 4.04 (q, 2H, OCH₂CH₃);
7.55-7.81 (4H, m, arom. H).

### c) Titelverbindung

durch Verseifen des Ethylesters mittels 2N NaOH in Ethanol.
Ausb. 81 % d.Th., Schmp. 139-141°C (60proz. Ethanol)

### 5) 5-[4-(2-(4-Chlor-benzolsulfonylamino)ethyl)cyclohexyl]pentansäure

über folgende Stufen:

### a) 5-[4-(2-Aminoethyl)cyclohexyl]pentansäureethylester- hydrochlorid

durch Hydrieren von 5-[4-(2-Aminoethyl) phenyl]pentansäure-ethylester-hydrochlorid an Platin in Eisessig.
Ausb. 57 % d.Th. Hydrochlorid. Wachsartige Substanz.

### b) 5-[4-(2-(4-Chlor-benzolsulfonylamino) ethyl)cyclohexyl]pentansäure-ethylester

durch Umsetzen des nach a) erhaltenen Produktes mit 4-Chlor-benzolsulfochlorid in Methylenchlorid in Gegenwart von Triethylamin.
Ausb. 72 % d.Th., farbloses Oel.
¹H-NMR (300 MHz, d₆-DMSO): δ = 1.17 (t, J = 7 Hz, 3H, OCH₂CH₃); 2.24 (t, J = 7 Hz, 2H, -CH₂COOEt); 4.04 (q, J = 7 Hz, 2H, OCH₂CH₃); 7.55 (t, J = 5Hz, 1H, -SO₂NH); 7.62-6.82 (m, 4H, arom. Protonen).

### c) Titelverbindung

durch Verseifen des Ethylesters mittels 2N NaOH in Methanol.
Ausb. 85 % d.Th., Schmp. 98-100°C.

### 6) 4-[2-(4-Brom-benzolsulfonylamino)ethyl]cyclohexylessigsäure

über folgende Stufen:

### a) 4-[2-4-Brom-benzolsulfonylamino)ethyl] cyclohexyl-essigsäure-ethylester

durch Umsetzen von 4-(2-Aminoethyl)cyclohexylessigsäure-ethylester-hydrochlorid mit 4-Brombenzolsulfochlorid in Methylenchlorid in Gegenwart von Triethylamin.
Ausb. 74 % d.Th., Schmp. 62°C.

### b) Titelverbindung

durch Verseifen des Ethylesters mit 2N NaOH in Ethanol.
Ausb. 85 % d.Th., Schmp. 124-126°C (Toluol).

### 7) 4-[2-(4-Methyl-benzolsulfonylamino)ethyl]cyclohexyl-essigsäure

über folgende Stufen:

### a) 4-[2-(4-Methyl-benzolsulfonylamino)ethyl] cyclohexyl-essigsäure-ethylester

durch Umsetzen von 4-(2-Aminoethyl)cyclohexylessigsäure-ethylesterhydrochlorid mit p-Toluolsulfochlorid in Methylenchlorid in Gegenwart von Triethylamin.
Ausb. 79 % d.Th., farbl. Oel. n = 1.5202
¹H-NMR (300 MHz, d₆-DMSO): δ = 2.12 und 2.20 (2d, J = 7 Hz, zus. 2H, -CH₂COOEt); 2.68-2.78 (m, 2H, NHCH₂); 7.30-7.70 (m, 5H, -NHSO₂ und arom. Protonen).

### b) Titelverbindung

durch Verseifen des Ethylesters mit 2N NaOH in Ethanol.
Ausb. 72 % d.Th., Schmp. 104-105°C (Toluol)

### 8) 4-[2-(4-Cyano-benzolsulfonylamino)ethyl]cyclohexyl-essigsäure-ethylester

durch Umsetzen von 4-Cyano-benzolsulfonylchlorid mit 4-(2-Aminoethyl)cyclohexylessigsäure-ethylesterhydrochlorid in Methylenchlorid in Gegenwart von Triethylamin.
Ausb. 95 % d.Th., farbloses Oel.
¹H-NMR (300 MHz, d₆-DMSO): δ = 2.12 und 2.21 (2d, J = 7 Hz, zus. 2H, CH₂COOEt); 2.75-2.85 (m, 2H, NHCH₂); 7.78 (t, J = 4 Hz, 1H, SO₂NH); 7.91-8.08 (m, 4H, arom. Protonen).

### 9) 4-[2-(4-Aminocarbonyl-benzolsulfonylamino)ethyl]cyclohexyl-essigsäure

durch Verseifen des nach Beispiel 2.8 erhaltenen 4-[2-(4-Cyano-benzolsulfonylamino)ethyl]cyclohexylessigsäure-ethylesters mit 2N NaOH in Ethanol in Analogie zu Beispiel 2 c.
Ausb. 72 % d.Th., Schmp. 172-174°C (Isopropanol).

### Beispiel 3

### cis- und trans-4-[2-(4-Chlor-benzolsulfonylamino)ethyl]cyclohexyloxyessigsäure

### a) cis- und trans-4-[2-(4-Chlor-benzolsulfonylamino)ethyl]cyclohexyloxyessigsäure-ethylester

Durch Umsetzen von cis/trans-4-(2-Aminoethyl)cyclohexyloxyessigsäure-ethylester mit 4-Chlor-benzolsulfonylchlorid in Analogie zu Beispiel 1 c erhält man in 95 % Ausbeute ein Gemisch aus 80 % cis- und 20 % trans-Verbindung. Schmp. 47-53°C.

Nach Trennung an einer präparativen Mitteldruck-Flüssigchromatographie-Säule (Lichroprep CN/Cyclohexan + Ether 1.7 + 1 Vol.)
erhält man
64 % d.Th. reines cis-Isomer, Schmp. 65°C und
9 % d.Th. reines trans-Isomer, Schmp. 75-77°C.
- cis:: ¹H-NMR (300 MHz, d₆-DMSO): δ = 1.19 (3H, CH₃); 1.1-1.4 (7H); 1.70 (2H, H eq.); 2.78 (2H, N-CH₂-); 3.50 (1H H eq.); 4.02 (2H, CH₂); 4.10 (H, OCH₂-); 7.56 (1H, NH); 7.65 (2H, H arom.); 7.78 (2H, H arom.).
- trans:: ¹H-NMR (300 MHz, d₆-DMSO): δ = 0.82 (2H, H ax.); 1.08 (3H, H ax.); 1.19 (3H, -CH₃); 1.25 (2H, CH₂); 1.59 (2H, H eq.); 1.93 (2H, H eq.); 2.77 (2H, N-CH₂); 3.19 (1H, H ax.); 4.05 (2H; CH₂); 4.10 (2H, 0-CH₂); 7.55 (1H, NH); 7.64 (2H, H arom.); 7.78 (2H, H arom.).

### b) cis- und trans-4-[2-(4-Chlor-benzolsulfonylamino)ethyl]cyclohexyloxyessigsäure

Man hält ein Gemisch aus 3.0 g (7.4 mmol) cis-Ester, 30 ml Ethanol und 12 ml 2N NaOH drei Stdn. auf 50°C und destilliert anschließend das Ethanol ab. Der Rückstand wird mit 20 ml Wasser verdünnt, dann säuert man durch tropfenweise Zugabe von 2N HCl an, saugt ab und trocknet.
Ausb. 2.6 g (93 % d.Th.) cis-Säure mit dem Schmp. 105-106°C (Essigester)
¹H-NMR (300 MHz, d₆-DMSO): δ = 1.1-1.4 (7H); 1.70 (2H, H eq.); 2.78 (2H, N-CH₂); 3.50 (1H, H eq.); 2.93 (2H, CH₂); 7.56 (1H, NH); 7.65 (2H, H arom.); 7.78 (2H, H arom.).

In analoger Weise erhält man aus dem trans-Ester die trans-Säure mit dem Schmp. 118-120°C.
¹H-NMR (300 MHz, d₆-DMSO): δ = 0.82 (2H, H ax.); 1.07 (2H, H ax.); 1.18 (1H, H ax.); 1.23 (2H, CH₂); 1.58 (2H, H eq.); 1.92 (2H, H eq.), 2.76 (2H, N-CH₂); 3.19 (1H, H ax.); 3.96 (2H, CH₂); 7.55 (1H, NH); 7.64 (2H, H arom.); 7.78 (2H, H arom.).

### Beispiel 4

### cis-4-[2-(4-Chlor-benzolsulfonylamino)ethyl]cyclohexyloxyessigsäure-(tetrazol-5-yl-amid)

Zu einer Lösung aus 2.0 g (5.3 mmol) cis-4-[2-(4-Chlor-benzolsulfonylamino)ethyl]cyclohexylessigsäure und 25 ml abs. THF tropft man 0.86 g (5.3 mmol) Carbonyl-bisimidazol, rührt 10 min nach, gibt 0.74 g (5.3 mmol) 4-Nitrophenol zu und rührt weitere 10 min bei 40°C. Dann werden 0.91 g (100 mmol) wasserfreies 5-Amino-1,2,3,4-tetrazol zugegeben. Man hält drei Stdn. bei 60°C, dampft i.Vak. ein und verrührt den Rückstand mit 0.5N HCl. Nach Absaugen und Waschen mit Wasser wird aus Ethanol umkristallisiert.
Ausb. 1.40 g (59 % d.Th.), Schmp. 191-192°C.

In analoger Weise erhält man das
trans-4-[2-(4-Chlor-benzolsulfonylamino)ethyl]cyclohexyloxyessigsäure-(tetrazol-5-yl-amid)
Ausb. 73 % d.Th., Schmp. 214°C (Ethanol).

### Beispiel 5

### trans-4-[2-(N-Benzoyl-4-chlor-benzolsulfonylamino)ethyl]cyclohexyloxyessigsäure-ethylester

Zu einem eiskalten Gemisch aus 4.0 g (10 mmol) trans-4-[2-(4-Chlor-benzolsulfonylamino)ethyl]cyclohexyloxyessigsäure-ethylester, 50 ml Methylenchlorid und 1.5 g (15 mmol) Triethylamin tropft man ein Gemisch aus 1.4 g (10 mmol) Benzoylchlorid und 30 ml Methylenchlorid. Man hält 4 Std. auf Rückflußtemperatur, kühlt ab, filtriert und wäscht das Filtrat mit verd. HCl und Wasser. Dann wird getrocknet (Na₂SO₄) und eingedampft. Nach Reinigung über Kieselgel/Cyclohexan + Ether (2 + 1 Vol.)
Ausb. 1.7 g (67 % d.Th.), Schmp. 81-82°C.

### Beispiel 6

### 4-[2-(4-Chlor-benzolsulfonylamino)ethyl]cyclohexylessigsäure-(tetrazol-5-yl-amid)

wird in Analogie zu Bsp. 4 aus 4-[2-(4-Chlor-benzolsulfonylamino)ethyl]cyclohexylessigsäure und 5-Amino-1,2,3,4-tetrazol dargestellt.
Zers. 216°C (Zers.) (Ethanol)

### Beispiel 7

### 1-[2-(4-Chlor-benzolsulfonylamino)ethyl]-4-(tetrazol-5-yl-methylenoxy)cyclohexan

### a) 4-[2-(4-Chlor-benzolsulfonylamino)ethyl]cyclohexyloxyessigsäureamid

Zu einem Gemisch aus 4.5 g (12 mmol) 4-[2-(4-Chlor-benzolsulfonylamino)ethyl]cyclohexyloxyessigsäure, 25 ml abs. THF und 1.43 g (14 mmol) Triethylamin tropft man bei -15°C langsam eine Lösung aus 1.33 g (12 mmol) Chlorameisensäure-ethylester und 10 ml abs. THF und läßt anschließend auf 20°C kommen. Dann wird abgesaugt und auf das Filtrat bei Raumtemperatur Ammoniak geleitet. Man rührt weitere zwei Stdn., filtriert, dampft das Filtrat ein und bringt durch Zusetzen von Isohexan zur Kristallisation.
Ausb. 4.3 g (96 % d.Th.), Schmp. 105-108°C.

### b) 4-[2-(4-Chlor-benzolsulfonylamino)ethyl]cyclohexyloxyacetonitril

Man hält ein Gemisch aus 4.0 g (10 mmol) des nach a) erhaltenen Carbonamids, 50 ml Toluol und 3.0 g (20 mmol) Phosphorpentoxid drei Std. auf 110°C und kühlt dann ab. Nach Zugabe von Wasser wird mit Essigester extrahiert und die organische Phase getrocknet (Na₂SO₄) und eingedampft.
Ausb. 2.8 g (74 % d.Th.), farbloses Oel.
¹H-NMR (300 MHz), d₆-DMSO): δ = 2.73-2.83 (m, 2H, NHCH₂); 4.39 (s, 2H, OCH₂CN); 7.55 (t, J = 6 Hz, 1H, NHSO₂); 7.63-7.83 (m, 4H, arom. Protonen).

### c) Titelverbindung

Ein Gemisch aus 2.5 g (7 mmol) des nach b) erhaltenen Nitrils, 70 ml 1-Methylpyrrolidon, 1.49 g (10 mmol) Triethylammoniumchlorid und 1.40 g (20 mmol) Natriumazid wird 6 Stdn. bei 150°C gerührt. Dann destilliert man das 1-Methylpyrrolidon i.Vak. ab, löst den Rückstand in verd. NaOH und schüttelt dreimal mit Ether aus. Die wäßrige Lösung wird angesäuert (6N HCl), dann viermal mit Essigester extrahiert. Man trocknet den Extrakt (Na₂SO₄) und dampft ein:
Ausb. 2.1 g (75 % d.Th.), Schmp. 122-123°C.

## Patentansprüche

1. Sulfonamide der allgemeinen Formel I 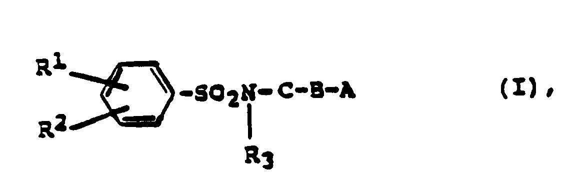 in welcher
R¹ und R² gleich oder verschieden sein können und jeweils Wasserstoff, ein Halogenatom, eine C₁-C₆-Alkyl-, Trifluormethyl-, Cyan-, Carboxyl-, C₁-C₆-Alkoxy-carbonyl-, Aminocarbonyl-, N-C₁-C₆-Alkyl-aminocarbonyl- oder N,N-Di-C₁-C₆-alkylamino-carbonylgruppe oder für den Fall, daß R₁ und R₂ zueinander o-ständige Alkylgruppen darstellen, R₁ und R₂ zusammen mit den beiden C-Atomen, an die sie geknüpft sind, einen gesättigten oder ungesättigten C₅-C₇-Alkylenring bilden,
R³ ein Wasserstoffatom, eine C₁-C₆-Alkyl-, eine durch ein Wasserstoffatom oder durch eine C₁-C₆-Alkyl-, Phenyl- oder Phenyl-C₁-C₆-alkylgruppe substituierte Carbonylgruppe, eine Phenyl-C₁-C₆-alkyl- oder Phenyl-C₂-C₆-alkenylgruppe darstellt, wobei die Phenylteile durch Halogen, C₁-C₆-Alkyl oder Trifluormethyl substituiert sein können,
C die Gruppe -(CH₂)ₘ- mit m = 0 bis 3 oder eine verzweigte C₂-C₅-Alkylengruppe bedeutet, wobei eine Methylengruppe -CH₂- der Gruppe C durch ein Sauerstoffatom, Schwefelatom oder durch die Hydroxymethylengruppe -CHOH- oder durch die Carbonylgruppe -CO- ersetzt sein kann,
B 1,2-, 1,3-, 1,4-Cyclohexyliden, 1,2- oder 1,3-Cyclopentyliden symbolisiert,
A eine C₁₋₆-Alkylkette, eine Hydroxy-C₁₋₆-alkylgruppe, Carboxy-, Carboxy-C₁₋₆-alkyl-, CarboxyC₁-C₆-alkoxy-, Tetrazolyl-, Tetrazolyl-C₁-C₆-alkyl, Tetrazolyl-C₁-C₆-alkoxy-, Tetrazolylaminocarbonyl-C₁-C₆-alkyl- oder eine Tetrazolylaminocarbonyl-C₁-C₆-alkoxy-Gruppe oder einen Rest D-R⁴ bedeutet, in dem D eine -CO-oder -CHOH-Gruppe und R⁴ Wasserstoff, einen C₁-C₅-Alkyl-, Hydroxy-C₁-C₅-alkyl-, Carboxy-, Carboxy-C₁-C₅-alkyl, Tetrazolyl-oder Tetrazolyl-C₁-C₅-alkylrest darstellt, wobei in allen Fällen, in denen A eine Carboxylgruppe enthält, diese durch die Hydroxamsäuregruppe -CO-N(OH)R₅ ersetzt sein kann, in der R₅ ein Wasserstoffatom oder eine C₁-C₅-Alkylgruppe bedeutet, und für den Fall, daß A eine Carboxy- und eine Hydroxygruppe enthält, gegebenenfalls deren Lactone,
sowie deren pharmakologisch verträgliche Salze, Ester und Amide, optisch aktive Formen sowie cis- und trans-Isomere.

2. Sulfonamide der Formel I gemäß Anspruch 1, dadurch gekennzeichnet, daß R¹ oder R² ein Wasserstoff- oder Halogenatom, eine C₁-C₆-Alkyl-, Trifluormethyl-, Cyanooder Aminocarbonylgruppe darstellt.

3. Sulfonamide der Formel I gemaß Anspruch 1 oder 2, dadurch gekennzeichnet, daß R₃ ein Wasserstoffatom, eine C₁-C₆-Alkyl-, C₁-C₆-Alkylcarbonyl-, Benzoyl-, Benzyl-, Phenethyl- oder Cinnamylgruppe bedeutet.

4. Sulfonamide der Formel I gemäß einem der Ansprüche 1 - 3, dadurch gekennzeichnet, daß C die Gruppe -(CH₂)ₘ- mit m = 2, 3 oder die Gruppe -O-CH₂-, -CH₂-CH₂-O-, -CH₂-CO- oder -CH₂-CHOH- bedeutet.

5. Sulfonamide der Formel I gemäß einem der Ansprüche 1 - 4, dadurch gekennzeichnet, daß B den 1,3- oder 1,4-Cyclohexylidenrest darstellt.

6. Sulfonamide der Formel I gemäß einem der Ansprüche 1 - 5, dadurch gekennzeichnet, daß A eine C₁-C₄-Alkyl-, eine C₁-C₄-Hydroxyalkyl-, Carboxy-, Carboxy-C₁-C₄-alkyl, Carboxy-C₁-C₄-alkoxy-, Tetrazolyl-C₁-C₄-alkyl-, Tetrazolyl-C₁-C₄-alkoxy-, Tetrazolylaminocarbonyl-C₁-C₃-alkyl- oder Tetrazolylaminocarbonyl-C₁-C₃-alkoxy-Gruppe, oder einen Rest D-R₄ bedeutet, in dem D eine -CO- oder -CHOH-Gruppe und R₄ ein Wasserstoffatom, eine C₁-C₃-Alkyl-, Hydroxy-C₁-C₄-alkyl- oder Carboxy-C₁-C₃-alkyl-gruppe bedeutet, wobei in allen Fällen, in denen A eine Carboxylgruppe enthält, diese durch die Hydroxamsäuregruppe -CON(OH)H ersetzt sein kann.

7. Sulfonamide der Formel I gemäß Anspruch 1 ausgewählt aus der Gruppe der folgenden Verbindungen:
4-[2-(Benzolsulfonylamino)ethyl]-cyclohexyloxyessigsäure
4-[2-(4-Chlor-benzolsulfonylamino)ethyl]cyclohexancarbonsäure
4-[2-(4-Chlor-benzolsulfonylamino)ethyl]cyclohexylessigsäure
4-[2-(4-Brom-benzolsulfonylamino)ethyl]cyclohexylessigsäure
cis- und trans-4-[2-(4-Chlor-benzolsulfonylamino) ethyl]-cyclohexyloxyessigsäure
cis- und trans-4-(2-(4-Chlor-benzolsulfonylamino)ethyl] cyclohexyl-oxy-essigsäure-(tetrazol-5-yl-amid)
trans-4-(2-(N-Benzoyl-4-chlor-benzolsulfonylamino) ethyl]-cyclohexyloxyessigsäure-ethylester
4-[2-(4-Chlor-benzolsulfonylamino)ethyl]cyclohexylessigsäure-(tetrazol-5-yl-amid)
1-[2-(4-Chlor-benzolsulfonylamino) ethyl]-4-(tetrazol-5-yl-methylenoxy)cyclohexan.

8. Verfahren zur Herstellung von Verbindungen der Formel I gemäß einem der Ausprüche 1-7, dadurch gekennzeichnet, daß man
a) ein Amin der allgemeinen Formel II 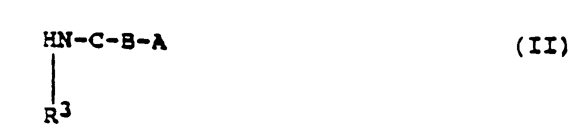 in welcher R³, m, A und B die angegebene Bedeutung haben, oder ein Säureadditionssalz des Amins in an sich bekannter Weise mit einer Sulfonsäure der allgemeinen Formel III 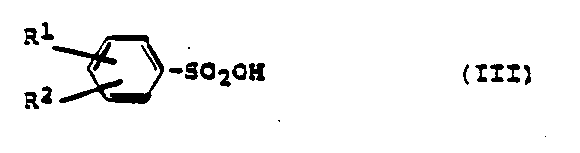 in welcher R¹ und R² die oben angegebene Bedeutung haben, bzw. mit einem Derivat derselben umsetzt,
oder
b) ein Sulfonamid der allgemeinen Formel IV 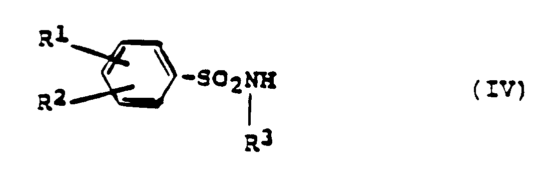 mit einer Verbindung der allgemeinen Formel V
X-C-B-A (V)
in der X eine reaktive Gruppe darstellt, und m, B und A die oben angegebene Bedeutung haben, zur Umsetzung bringt, oder für den Fall, daß A eine Hydroxygruppe enthält, ein Sulfonamid der allgemeinen Formel IV mit einer Verbindung der allgemeinen Formel V, in der A anstelle der Hydroxygruppe eine Oxogruppe enthält, zur Umsetzung bringt, und anschließend die Oxogruppe zur Hydroxygruppe reduziert,
oder
c) eine Verbindung der allgemeinen Formel Ia 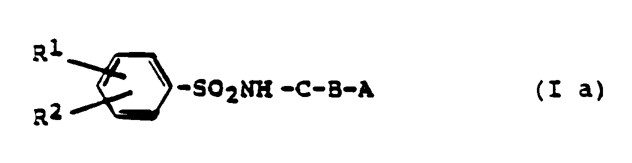 in der R₁, R₂, m, B und A die oben angegebenen Bedeutungen haben, mit einer Verbindung der allgemeinen Formel VI
R³-X (VI)
in der R³ die oben angegebene Bedeutung besitzt, jedoch kein Wasserstoffatom sein kann, und X ein leicht abspaltbarer Rest ist, umsetzt, oder für den Fall, daß A eine Hydroxgruppe enthält, eine Verbindung der allgemeinen Formel Ia, in der A anstelle der Hydroxygruppe eine Oxogruppe enthält, mit einer Verbindung der allgemeinen Formel VI umsetzt, und anschließend die Oxogruppe zur Hydroxygruppe reduziert,
und gegebenenfalls die so erhaltenen Verbindungen der allgemeinen Formel I nachträglich in andere Verbindungen der allgemeinen Formel I umwandelt oder in die pharmakologisch verträglichen Salze überführt.

9. Arzneimittel enthaltend mindestens ein Sulfonamid wie in einem der Ansprüche 1 - 7 angegeben sowie pharmazeutisch übliche Hilfs- oder Trägerstoffe.

10. Verwendung von Sulfonamiden wie in den Ansprüchen 1 - 7 angegeben zur Herstellung von Arzneimitteln zur Behandlung von Herz- und Kreislauferkrankungen, Nierenfunktionsstörungen, Asthma, thrombotischen Erkrankungen und cerebralen ischämischen Zuständen.

11. Verfahren zur Herstellung von Arzneimitteln enthaltend mindestens eine Verbindung der Formel I wie in den Ansprüchen 1 - 7 angegeben, dadurch gekennzeichnet, daß man eine Verbindung der Formel I zusammen mit üblichen pharmazeutischen Hilfs- oder Trägerstoffen in eine geeignete galenische Darreichungsform bringt.

## Claims

1. Sulphonamides of the general formula I 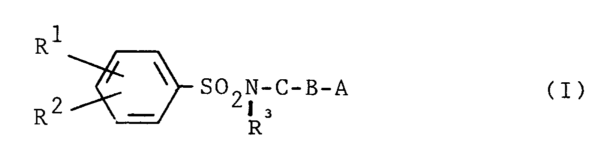 in which R¹ and R² can be the same or different and in each case represent hydrogen, a halogen atom, a C₁-C₆-alkyl, trifluoromethyl, cyano, carboxyl, C₁-C₆-alkoxycarbonyl, aminocarbonyl, N-C₁-C₆-alkylaminocarbonyl or N,N-di-C₁-C₆-alkylaminocarbonyl group or for the case that R¹ and R² represent alkyl groups o-standing to one another, R¹ and R², together with the two C-atoms to which they are attached, form a saturated or unsaturated C₅-C₇-alkylene ring, R³ represents a hydrogen atom, a C₁-C₆-alkyl, a carbonyl group substituted by a hydrogen atom or by a C₁-C₆-alkyl, phenyl or phenyl-C₁-C₆-alkyl group, a phenyl-C₁-C₆-alkyl or phenyl-C₂-C₆-alkenyl group, whereby the phenylmoieties can be substituted by halogen, C₁-C₆-alkyl or trifluoromethyl, C signifies the group -(CH₂)ₘ- with m = 0 to 3 or a branched C₂-C₅-alkylene group, whereby a methylene group -CH₂- of the group C can be replaced by an oxygen atom, sulphur atom or by the hydroxymethylene group -CHOH- or by the carbonyl group -CO-, B symbolises 1,2-, 1,3-, 1,4-cyclohexylidene, 1,2- or 1,3-cyclopentylidene, A signifies a C₁-C₆-alkyl chain, a hydroxy-C₁-C₆-alkyl group, carboxyl, carboxy-C₁-C₆-alkyl, carboxy-C₁-C₆-alkoxy, tetrazolyl, tetrazolyl-C₁-C₆-alkyl, tetrazolyl-C₁-C₆-alkoxy, tetrazolylaminocarbonyl-C₁-C₆-alkyl or a tetrazolylaminocarbonyl-C₁-C₆-alkoxy group or a radical -D-R⁴, in which D represents a -CO- or -CHOH- group and R⁴ hydrogen, a C₁-C₅-alkyl, hydroxy-C₁-C₅-alkyl, carboxyl, carboxy-C₁-C₅-alkyl, tetrazolyl or tetrazolyl-C₁-C₅-alkyl radical, whereby, in all cases in which A contains a carboxyl group, this can be replaced by the hydroxamic acid group -CO-N(OH)R⁵, in which R⁵ signifies a hydrogen atom or a C₁-C₅-alkyl group and for the case that A contains a carboxyl and a hydroxyl group, possibly their lactones, as well as their pharmacologically compatible salts, esters and amides, optically-active forms, as well as cis and trans isomers.

2. Sulphonamides of the formula I according to claim 1, characterised in that R¹ or R² represents a hydrogen or halogen atom, a C₁-C₆-alkyl, trifluoromethyl, cyano or aminocarbonyl group.

3. Sulphonamides of the formula I according to claim 1 or 2, characterised in that R³ signifies a hydrogen atom, a C₁-C₆-alkyl, C₁-C₆-alkylcarbonyl, benzoyl, benzyl, phenethyl or cinnamyl group.

4. Sulphonamides of the formula I according to one of claims 1 - 3, characterised in that C signifies the group -(CH₂)ₘ- with m = 2, 3 or the group -O-CH₂-, -CH₂-CH₂-O-, -CH₂-CO- or -CH₂-CHOH- radical.

5. Sulphonamides of the formula I according to one of claims 1 - 4, characterised in that B represents the 1,3- or 1,4-cyclohexylidene radical.

6. Sulphonamides of the formula I according to one of claims 1 - 5, characterised in that A signifies a C₁-C₄-alkyl, a C₁-C₄-hydroxyalkyl, carboxyl, carboxy-C₁-C₄-alkyl, carboxy-C₁-C₄-alkoxy, tetrazolyl-C₁-C₄-alkyl, tetrazolyl-C₁-C₄-alkoxy, tetrazolylaminocarbonyl-C₁-C₃-alkyl or tetrazolylaminocarbonyl-C₁-C₃-alkoxy group or a radical -D-R⁴, in which D signifies a -CO- or -CHOH- group and R⁴ a hydrogen atom, a C₁-C₃-alkyl, hydroxy-C₁-C₄-alkyl or carboxy-C₁-C₃-alkyl group, whereby, in all cases in which A contains a carboxyl group, this can be replaced by the hydroxamic acid group -CON(OH)H.

7. Sulphonamides of the formula I according to claim 1 selected from the group of the following compounds:
4-[2-(benzenesulphonylamino)-ethyl]-cyclohexyloxyacetic acid
4-[2-(4-chlorobenzenesulphonylamino)-ethyl]-cyclohexane-carboxylic acid
4-[2-(4-chlorobenzenesulphonylamino)-ethyl]-cyclohexylacetic acid
4-[2-(4-bromobenzenesulphonylamino)-ethyl]-cyclohexylacetic acid
cis- and trans-4-[2-(4-chlorobenzenesulphonylamino)-ethyl]-cyclohexyloxyacetic acid
cis- and trans-4-[2-(4-chlorobenzenesulphonylamino)-ethyl]-cyclohexyloxyacetic acid (tetrazol-5-ylamide) trans-4-[2-(N-bensoyl-4-chlorobenzenesulphonylamino)-ethyl]-cyclohexyloxyacetic acid ethyl ester
4-[2-(4-chlorobenzenesulphonylamino)-ethyl]-cyclohexylacetic acid (tetrazol-5-ylamide)
1-[2-(4-chlorobenzenesulphonylamino)-ethyl]-4-(tetrazol-5-yl-methyleneoxy)-cyclohexane.

8. Process for the preparation of compounds of the formula I according to one of claims 1 - 7, characterised in that one
a) reacts an amine of the general formula II 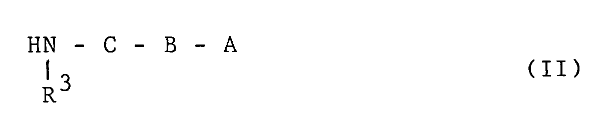 in which R³, m, A and B have the given meaning, or an acid-addition salt of the amine, in per se known manner with a sulphonic acid of the general formula III 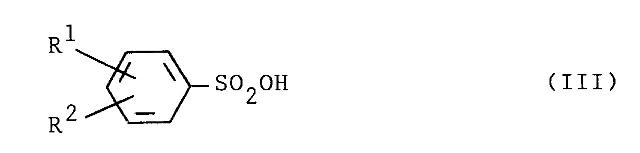 in which R¹ and R² have the above-given meaning, or with a derivative thereof; or
b) brings a sulphonamide of the general formula IV 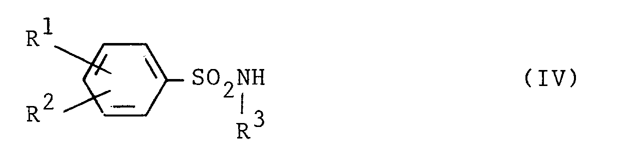 to reaction with a compound of the general formula V
X - C - B - A (V)
in which X represents a reactive group and m, B and A have the above-given meaning, or, for the case that A contains a hydroxyl group, brings a sulphonamide of the general formula IV to reaction with a compound of the general formula V in which A contains an oxo group instead of the hydroxyl group and subsequently reduces the oxo group to the hydroxyl group; or
c) reacts a compound of the general formula Ia 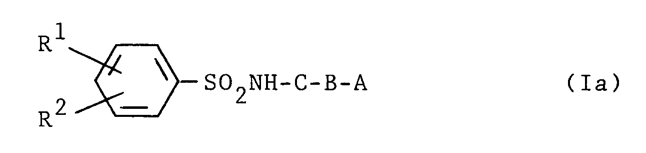 in which R¹, R², m, B and A have the above-given meaning, with a compound of the general formula VI
R³-X (VI)
in which R³ possesses the above-given meaning but cannot be a hydrogen atom and X is a residue which can easily be split off or, for the case that A contains a hydroxyl group, reacts a compound of the general formula Ia, in which A contains an oxo group instead of the hydroxyl group, with a compound of the general formula VI and subsequently reduces the oxo group to the hydroxyl group,
and possibly subsequently converts the so-obtained compounds of the general formula I into other compounds of the general formula I and/or converts into the pharmacologically compatible salts.

9. Medicaments containing at least one sulphonamide as given in one of claims 1 - 7, as well as pharmaceutically usual adjuvant or carrier materials.

10. Use of sulphonamides as given in claims 1 - 7 for the preparation of medicaments for the treatment of heart and circulatory diseases, functional disturbances of the kidneys, asthma, thrombotic diseases and cerebral ischaemic states.

11. Process for the preparation of medicaments containing at least one compound of the formula I as given in claims 1 - 7, characterised in that one brings a compound of the formula I, together with usual pharmaceutical adjuvant or carrier materials, into a suitable galenical form of administration.

## Revendications

1. Sulfonamide de formule générale I 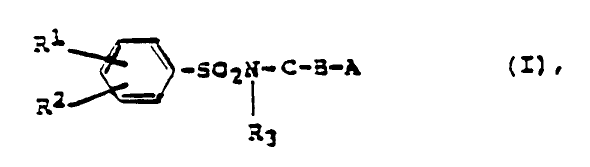 dans laquelle
R¹ et R² peuvent être identiques ou différents et représentent chacun un atome d'hydrogène, un atome d'halogène, un groupe alkyle en C₁-C₆, trifluorométhyle, cyano, carboxyle, alcoxy(C₁-C₆)carbonyle, aminocarbonyle, N-alkyl(C₁-C₆)aminocarbonyle ou N,N-dialkyl(C₁-C₆)aminocarbonyle, ou dans le cas où R₁ et R₂ représentent des groupes alkyle en position ortho l'un par rapport à l'autre, R₁ et R₂ forment, conjointement avec les deux atomes de C auxquels ils sont liés, un noyau alkylène en C₅-C₇ saturé ou insaturé,
R³ représente un atome d'hydrogène, un groupe alkyle en C₁-C₆, un groupe carbonyle substitué par un atome d'hydrogène ou par un groupe alkyle en C₁-C₆, phényle ou phényl-alkyle(C₁-C₆), un groupe phényl-alkyle(C₁-C₆) ou phényl-alcényle(C₂-C₆), la partie phényle pouvant être substituée par un atome d'halogène, un groupe alkyle en (C₁-C₆) ou un groupe trifluorométhyle,
C représente le groupe -(CH₂)m-, avec m=0 à 3, ou un groupe alkylène en C₂-C₅ ramifié, un groupe méthylène -CH₂- du groupe C pouvant être substitué par un atome d'oxygène, un atome de soufre ou le groupe hydroxyméthylène -CHOH- ou par le groupe carbonyle -CO-,
B représente un groupe 1,2-, 1,3-, 1,4-cyclohexylidène, 1,2- ou 1,3-cyclopentylidène,
A représente une chaîne alkyle en C₁-C₆, un groupe hydroxyalkyle en C₁-C₆, un groupe carboxyle, un groupe carboxyalkyle en C₁-C₆, un groupe carboxyalcoxy(C₁-C₆), un groupe tétrazolyle, tétrazolyl-alkyle(C₁-C₆), tétrazolylalcoxy(C₁-C₆), tétrazolyl-aminocarbonyl-alkyle(C₁-C₆) ou un groupe tétrazolyl-aminocarbonyl-alcoxy(C₁-C₆) ou un reste D-R⁴ dans lequel D représente un groupe -CO- ou -CHOH- et R⁴ représente un atome d'hydrogène, un groupe alkyle en C₁-C₅, hydroxy-alkyle en C₁-C₅, carboxyle, carboxy-alkyle(C₁-C₅), tétrazolyle ou tétrazolyl-alkyle(C₁-C₅), étant entendu que dans tous les cas où A contient un groupe carboxyle, celui-ci peut être substitué par un groupe acide hydroxamique -CO-N(OH)R₅ dans lequel R₅ représente un atome d'hydrogène ou un groupe alkyle en C₁-C₅, et dans le cas où A contient un groupe carboxyle et un groupe hydroxy, éventuellement leur lactone,
ainsi que leurs sels, esters et amides pharmacologiquement acceptables, leurs formes optiquement actives ainsi que leurs isomères cis et trans.

2. Sulfonamide de formule I selon la revendication 1, caractérisé en ce que R¹ ou R² représente un atome d'hydrogène ou d'halogène, un groupe alkyle en C₁-C₆, trifluorométhyle, cyano ou aminocarbonyle.

3. Sulfonamide de formule I selon la revendication 1 ou 2, caractérisé en ce que R₃ représente un atome d'hydrogène, un groupe alkyle en C₁-C₆, alkyl-carbonyle(C₁-C₆), benzoyle, benzyle, phénéthyle ou cinnamyle.

4. Sulfonamide de formule I selon l'une quelconque des revendications 1-3, caractérisé en ce que C représente le groupe -(CH₂)ₘ- dans lequel m=2, 3, ou le groupe -O-CH₂-, -CH₂-CH₂-O-, -CH₂-CO- ou -CH₂-CHOH-.

5. Sulfonamide de formule I selon l'une quelconque des revendications 1-4, caractérisé en ce que B représente le groupe 1,3- ou 1,4-cyclohexylidène.

6. Sulfonamide de formule I selon l'une quelconque des revendications 1-5, caractérisé en ce que A représente un groupe alkyle en C₁-C₄, hydroxy-alkyle(C₁-C₄), carboxyle, carboxy-alkyle(C₁-C₄), carboxy-alcoxy(C₁-C₄), tétrazolyl-alkyle(C₁-C₄), tétrazolyl-alcoxy(C₁-C₄), tétrazolylaminocarbonyl-alkyle(C₁-C₃) ou tétrazolylaminocarbonylalcoxy(C₁-C₃), ou un reste D-R₄ dans lequel D représente un groupe -CO- ou -CHOH- et R₄ représente un atome d'hydrogène, un groupe alkyle en C₁-C₃, hydroxy-alkyle en C₁-C₄ ou carboxy-alkyle(C₁-C₃), étant entendu que dans tous les cas où A contient un groupe carboxyle, celui-ci peut être remplacé par le groupe acide hydroxamique -CON(OH)H-.

7. Sulfonamide de formule I selon la revendication 1, choisi dans le groupe des composé suivants:
acide 4-[2-(benzènesulfonylamino)éthyl]-cyclohexyloxy acétique
acide 4-[2-(4-chlorobenzènesulfonylamino)éthyl]cyclohexanecarboxylique
acide 4-[2-(4-chlorobenzènesulfonylamino)éthyl]cyclohexylacétique
acide 4-[2-(4-bromobenzènesulfonylamino)éthyl]cyclohexylacétique
acides cis- et trans-4-[2-(4-chlorobenzènesulfonylamino)-éthyl]cyclohexyloxyacétique
tétrazol-5-yl-amide des acides cis- et trans-4-[2-(4-chlorobenzènesulfonylamino)éthyl]cyclohexyl-oxyacétique
trans-4-[2-(N-benzoyl-4-chlorobenzènesulfonylamino)éthyl]-cyclohexyloxyacétate d'éthyle
tétrazol-5-yl-amide de l'acide 4-[2-(4-chlorobenzènesulfonylamino)éthyl]cyclohexylacétique
1-[2-(4-chlorobenzènesulfonylamino)éthyl]-4-(tétrazol-5-yl-méthylènoxy)cyclohexane.

8. Procédé de préparation des composés de formule I selon l'une quelconque des revendications 1 à 7, caractérisé en ce que
a) on fait réagir une amine de formule générale II 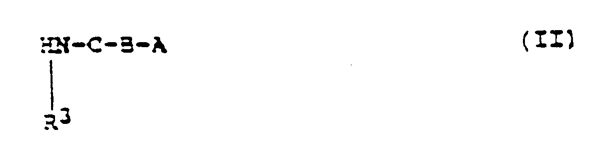 dans laquelle R³, m, A et B ont les significations indiquées plus haut, ou un sel d'addition d'acide de l'amine, de manière connue en soi, avec un acide sulfonique de formule générale III 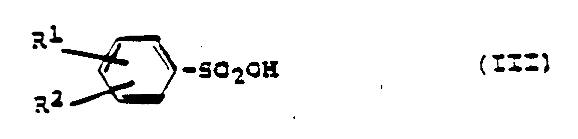 dans laquelle R¹ et R² ont les significations mentionnées plus haut, ou avec un dérivé de celui-ci,
ou
b) on fait réagir un sulfonamide de formule générale IV 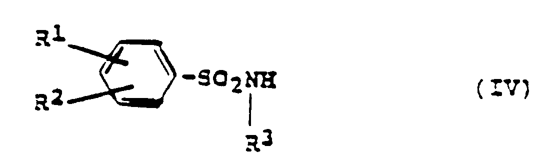 avec un composé de formule générale V
X-C-B-A (V)
dans laquelle X représente un groupe réactif et m, B et A ont les significations mentionnées ci-dessus, ou dans le cas où A contient un groupe hydroxy, on fait réagir un sulfonamide de formule générale IV avec un composé de formule générale V, dans lequel A contient un groupe oxo à la place du groupe hydroxy, et ensuite, on réduit le groupe oxo en groupe hydroxy,
ou
c) on fait réagir un composé de formule générale la 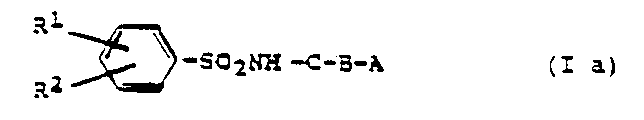 dans laquelle R¹, R², m, B et A ont les significations mentionnées ci-dessus, avec un composé de formule générale VI
R³-X (VI)
dans laquelle R³ a la signification mentionnée, mais ne peut toutefois représenter un atome d'hydrogène, et X représente un groupe facilement séparable, ou dans le cas où A contient un groupe hydroxy, on fait réagir un composé de formule générale la dans laquelle A contient un groupe oxo à la place du groupe hydroxy, avec un composé de formule générale VI et ensuite, on réduit le groupe oxo en groupe hydroxy,
et éventuellement, on transforme ultérieurement les composés ainsi obtenus, de formule générale I, en d'autres composés de formule générale I ou en leurs sels pharmacologiquement acceptables.

9. Médicament contenant au moins un sulfonamide selon l'une quelconque des revendications 1 à 7, ainsi que des adjuvants ou véhicules pharmaceutiques usuels.

10. Utilisation des sulfonamides selon les revendications 1-7, pour la préparation de médicaments destinés au traitement des maladies cardio-vasculaires, des perturbations de la fonction rénale, de l'asthme, des affections thrombotiques et des états ischémiques cérébraux.

11. Procédé de préparation de médicaments contenant au moins un composé de formule I selon la revendication 1-7, caractérisé en ce que l'on transforme un composé de formule I, conjointement avec des adjuvants ou véhicules pharmaceutiquement acceptables, en une forme galénique appropriée.
